# EUROPEAN PATENT APPLICATION

(11) **EP 4 793 281 A1**
(43) Date of publication of application: **19.08.2026**
(21) Application number: 24876658.6
(22) Date of filing: 11.10.2024
(51) Int. Cl.: C07K 14/47, C12N 15/12, C12Q 1/6883, C12N 15/11, A61K 38/17

(54) **ORGAN FIBROSIS-RELATED MICROPEPTIDE (OFMP) AND USE THEREOF**

(30) Priority: 13.10.2023 CN 202311332361
(71) Applicant: Nanjing Anji Biological Technology Co., Ltd., Nanjing, Jiangsu 210033 (CN)
(72) Inventor: XU, Hanmei, Nanjing, Jiangsu 211100 (CN); LI, Bangjie, Nanjing, Jiangsu 211100 (CN)
(74) Representative: Alpspitz IP
(86) International application number: PCT/CN2024/124285
(87) International publication number: WO 2025/077853

(57) **Abstract**

Provided are an organ fibrosis-related micropeptide (OFMP) and a use thereof. The OFMP is a stable translation product coded by an open reading frame (ORF) in a long non-coding RNA and having endogenous biological activity. It is found for the first time that the long non-coding RNA coding the micropeptide has an important relationship with organ fibrosis and can code the micropeptide. A synthetic peptide prepared from the OFMP has a significant inhibitory effect on a plurality of organ fibrosis key indicators, comprising a collagen type I α1 chain gene, α-smooth muscle actin, a transforming growth factor β1, and a connective tissue growth factor, indicating that the OFMP, a nucleotide sequence (ORF) for coding the micropeptide, and a long non-coding RNA sequence containing the nucleotide sequence have important clinical diagnosis and clinical treatment value on organ fibrosis.

## Description

### TECHNICAL FIELD

The present application pertains to the field of biomedicine and particularly relates to an organ fibrosis-related micropeptide (OFMP) and a use thereof.

### BACKGROUND ART

Long non-coding RNA (lncRNA) refers to a functional RNA molecule that comprises more than 200 nucleotides and is believed to be uncapable of being translated into proteins. Previous studies have shown that lncRNAs play significant roles in epigenetic regulation, cell cycle and differentiation regulation, and involve in major human diseases such as cancers and degenerative neurological diseases. With the rapid development of molecular biology, computational biology and deep sequencing technologies, a large number of previously unannotated non-classical short open reading frames (ORFs) hidden within lncRNAs have actually demonstrated the ability to code functional proteins. Micropeptides are translation products that are coded by short open reading frames (sORFs) and have a length of 2~100 codons.

Organ fibrosis refers to a fibrotic disease that may occur in various organs. The main pathological changes are an increase in fibrous connective tissue within the organs and tissues, and a decrease in parenchymal cells. The continuous fibrotic lesions occurring in organs may damage the structure and function of the organs, further leading to organ failure and the development of malignant tumors. They are regarded as tumor-like lesions that lie between benign and malignant, posing a serious threat to human health and life.

Discovering and studying an organ fibrosis-related micropeptide (OFMP) is of great significance for carrying out novel and effective disease diagnosis and treatment, and can provide important references and development values for exploring new application fields. However, there are currently no reports on micropeptides in the field of organ fibrosis research.

### SUMMARY OF THE INVENTION

The first objective of the present invention is to provide an organ fibrosis-related micropeptide (OFMP), wherein the amino acid sequence of the micropeptide is any of the following ones:
(a) an amino acid sequence shown in any of SEQ ID NO: 1-3;
(b) an amino acid sequence that has a homology of more than 85% with the amino acid sequence shown in any of SEQ ID NO: 1-3;
(c) an amino acid sequence formed by replacing, deleting or adding one or several amino acid residues in the amino acid sequence shown in any of SEQ ID NO: 1-3, which has the same function as the original sequence.

The micropeptide in the present invention is a stable translation product coded by a short open reading frame (sORF) in a long non-coding RNA (lncRNA) and having endogenous biological activity. In the present invention, this type of micropeptides is uniformly named Organ Fibrosis-related MicroPeptides (OFMP) based on the discovered functions. In the following text, the micropeptides related to SEQ ID NO: 1-3 are respectively named as OFMP3178, OFMP2067 and OFMP1080.

The second objective of the present invention is to provide a nucleotide sequence coding the foregoing micropeptide. This nucleotide sequence can be an lncRNA molecule that codes the foregoing polypeptide, or an ORF that the lncRNA molecule contains, or any other nucleotide sequence that can code the same amino acid sequence.

The third objective of the present invention is to provide a use of the foregoing micropeptide in preparing reagents or drugs for detecting or treating organ fibrosis. The micropeptide is used in the form of amino acids or nucleotides.

The fourth objective of the present invention is to provide a use of lncRNA in preparing reagents or drugs for detecting or treating organ fibrosis. The ID number of the lncRNA in the Ensembl database is: ENSG00000224023, ENSG00000263126 or ENSG00000265666.

As a preferred embodiment, the organ fibrosis includes one or more of liver fibrosis, renal fibrosis, pulmonary fibrosis, heart fibrosis, and skin fibrosis.

As a preferred embodiment, the detection of organ fibrosis is achieved through specific primers designed for the nucleotide sequences coding the foregoing micropeptide or the foregoing lncRNA of the nucleotide sequences.

The fifth objective of the present invention is to provide a recombinant vector containing the nucleotide sequence coding the foregoing micropeptide or the nucleotide sequence of the foregoing lncRNA. The recombinant vector can be a plasmid or a viral vector.

The sixth objective of the present invention is to provide a pharmaceutical composition. The pharmaceutical composition contains the foregoing micropeptide, or the nucleotide sequence coding the foregoing micropeptide or the nucleotide sequence of the foregoing lncRNA, and a pharmaceutically acceptable carrier thereof. The pharmaceutical composition can be used for preventing or treating organ fibrosis.

The seventh objective of the present invention is to provide an organ fibrosis detection kit. The kit contains specific primer pairs and/or antibodies designed for the nucleotide sequence coding the foregoing micropeptide or the nucleotide sequence of the foregoing lncRNA.

As a preferred embodiment, the nucleotide sequences of the specific primer pairs are selected from any one of the following groups:
(a) F: AGTGCCCGGACTTTTGTTCT; R: TGGGACTTTCACTCGGGGTA;
(b) F: ACCACATGGGCTCCGTTATG; R: GCTGCTTTGCCAAGTGAGTT;
(c) F: AGACACCGGCTTGTGTCATT; R: TCTGCCTGTGTTTGTGCCA.

The present invention has the following advantages:
(1) The present invention, by analyzing a plurality of organ fibrosis sequencing data, discovered for the first time that a plurality of lncRNA molecules had significant relationships with organ fibrosis, including ENSG00000224023, ENSG00000263126 and ENSG00000265666. Specifically, they are underexpressed in various kinds of cells and/or tissues of organ fibrosis and can be used for diagnosis or assistant diagnosis, treatment or assistant treatment of organ fibrosis, or for preparing drugs or reagents for diagnosis or assistant diagnosis, treatment or assistant treatment of organ fibrosis.
(2) The present invention discovered that the foregoing lncRNAs had open reading frames (ORFs) capable of coding micropeptides. The applicant discovered by analyzing the translation characteristics of the ORFs of the foregoing lncRNA molecules that they had the potential to code functional proteins. The applicant also conducted endogenous coding verification by such methods as gene editing, immunoprecipitation and LC-MS/MS, and confirmed that the foregoing lncRNA molecules had coding sequences (ORFs), which have not been reported in any previous studies. Based on the nucleotide sequences of the ORFs of ENSG00000224023, ENSG00000263126 and ENSG00000265666 (as shown in SEQ ID NO: 4-6), the present invention coded and obtained a series of novel micropeptides OFMP3178, OFMP2067 and OFMP1080. After searching the database (BLAST, UniProt) and the literature, no protein or peptide fragments with homologous sequences to the foregoing micropeptide were found. In other words, the micropeptide is a novel endogenous polypeptide in the human body, which was discovered for the first time. The nucleotide sequences coding micropeptides can be used for diagnosis or assistant diagnosis, treatment or assistant treatment of organ fibrosis, or for preparing reagents for diagnosis or assistant diagnosis, treatment or assistant treatment of organ fibrosis.
(3) The present invention provides novel micropeptides OFMP3178, OFMP2067 and OFMP1080 with novel amino acid sequences. Such micropeptides are stable translation products coded by ORF in lncRNA and having endogenous biological activity. The synthetic peptide of the novel micropeptides exhibits significant inhibitory effects in vitro on the key indicators of human liver fibrosis, renal fibrosis and pulmonary fibrosis, including a collagen type I αl chain gene (Col1a1), α-smooth muscle actin (α-SMA), a transforming growth factor β1 (TGF β1), and a connective tissue growth factor (CTGF). The foregoing novel micropeptides can also be used as drugs for treating fibrosis in other organs, significantly expanding the treatment spectrum of micropeptides and providing new ideas for the development of drugs for organ fibrosis.
(4) Based on the amino acid sequences of the foregoing novel micropeptides, the present invention obtained a synthetic peptide of the micropeptides by the solid-phase synthesis method; by constructing three organ fibrosis cell models, such as adding 10nmol of recombinant human TGFβ1 stimulating factor and different concentrations of synthetic peptides to human hepatic stellate cells LX-2, human non-small cell lung cancer cell line A549, and human proximal tubular cell HK2, respectively, co-incubating them for 24 h, extracting and collecting the total RNA of the cell samples with a TRIzol reagent, then reverse-transcribing the extracted total RNA using a reverse transcription reagent, and detecting the mRNA expression levels of genes Colla1 and α-SMA in the three organ fibrosis cell models by the real-time fluorescence quantitative PCR method. The results show that the synthetic peptides of micropeptides OFMP3178, OFMP2067 and OFMP1080 exhibited significant inhibitory effects on the three organ fibrosis cell models.

### BRIEF DESCRIPTION OF THE DRAWINGS

FIG. 1 shows the volcano plot for analyzing the low expression of lncRNA in organ fibrosis by the differential analysis method.
FIG. 2 shows a Sanger sequencing diagram of monoclonal cells of micropeptide OFMP3178 inserted with Flag tags by the gene editing method.
FIG. 3 shows a Sanger sequencing diagram of monoclonal cells of micropeptide OFMP2067 inserted with Flag tags by the gene editing method.
FIG. 4 shows a Sanger sequencing diagram of monoclonal cells of micropeptide OFMP1080 inserted with Flag tags by the gene editing method.
FIG. 5 shows the expression level of the target band of micropeptide OFMP3178 detected by the immunoprecipitation method.
FIG. 6 shows the expression level of the target band of micropeptide OFMP2067 detected by the immunoprecipitation method.
FIG. 7 shows the expression level of the target band of micropeptide OFMP1080 detected by the immunoprecipitation method.
FIG. 8 shows the protein spectrum of micropeptide OFMP3178 detected by the LC-MS/MS method.
FIG. 9 shows the protein spectrum of micropeptide OFMP2067 detected by the LC-MS/MS method.
FIG. 10 shows the protein spectrum of micropeptide OFMP1080 detected by the LC-MS/MS method.
FIG. 11 shows the melting curves for the specificity of four organ fibrosis gene primers detected by the qPCR method.
FIG. 12 shows using the qPCR method to detect genetic indicators constructed by a human liver fibrosis cell model.
FIG. 13 shows using the qPCR method to detect genetic indicators constructed by a human renal fibrosis cell model.
FIG. 14 shows using the qPCR method to detect genetic indicators constructed by a human pulmonary fibrosis cell model.
FIG. 15 shows the liquid chromatography result of a synthetic peptide of micropeptide OFMP3178.
FIG. 16 shows the liquid chromatography result of a synthetic peptide of micropeptide OFMP2067.
FIG. 17 shows the liquid chromatography result of a synthetic peptide of micropeptide OFMP1080.
FIG. 18 shows the mass spectrometry result of a synthetic peptide of micropeptide OFMP3178.
FIG. 19 shows the mass spectrometry result of a synthetic peptide of micropeptide OFMP2067.
FIG. 20 shows the mass spectrometry result of a synthetic peptide of micropeptide OFMP1080.
FIG. 21 shows using the qPCR method to detect the inhibitory effect of micropeptide OFMP3178 in a human liver fibrosis cell model.
FIG. 22 shows using the qPCR method to detect the inhibitory effect of micropeptide OFMP2067 in a human liver fibrosis cell model.
FIG. 23 shows using the qPCR method to detect the inhibitory effect of micropeptide OFMP1080 in a human liver fibrosis cell model.
FIG. 24 shows using the qPCR method to detect the inhibitory effect of micropeptide OFMP3178 in a human renal fibrosis cell model.
FIG. 25 shows using the qPCR method to detect the inhibitory effect of micropeptide OFMP2067 in a human renal fibrosis cell model.
FIG. 26 shows using the qPCR method to detect the inhibitory effect of micropeptide OFMP1080 in a human renal fibrosis cell model.
FIG. 27 shows using the qPCR method to detect the inhibitory effect of micropeptide OFMP3178 in a human pulmonary fibrosis cell model.
FIG. 28 shows using the qPCR method to detect the inhibitory effect of micropeptide OFMP2067 in a human pulmonary fibrosis cell model.
FIG. 29 shows using the qPCR method to detect the inhibitory effect of micropeptide OFMP1080 in a human pulmonary fibrosis cell model.

### DETAILED DESCRIPTION

The present application will be further described below in conjunction with embodiments.

Unless otherwise defined, all technical and scientific terms used herein have the same meanings as those commonly understood by those skilled in the technical field of the present application; the term "and/or" as used herein includes any and all combinations of one or more relevant listed items.

If the specific conditions are not specified in the embodiments, the conventional conditions or the conditions recommended by the manufacturer shall be complied with. The reagents or instruments used without indication of manufacturers are all conventional products that can be purchased from the market.

As used herein, the term "approximately" is used to provide flexibility and inaccuracy associated with a given term, measure or value. Those skilled in the art can readily determine the degree of flexibility of specific variables.

As used herein, the term "at least one of" is intended to be synonymous with "one or more of". For example, "at least one of A, B, and C" expressly includes only A, only B, only C, and combinations thereof.

Concentration, quantity and other numerical data can be presented in a range format herein. It should be understood that such a range format is merely used for convenience and simplicity, and it should be interpreted flexibly to not only include the numerical values explicitly stated as the limits of the range, but also include all individual numerical values or sub-ranges in the range, as if each numerical value and sub-range are explicitly stated. For example, the numerical range of approximately 1 to approximately 4.5 should be interpreted as not only including the explicitly stated limits of 1 to approximately 4.5, but also including individual numbers (such as 2, 3, 4) and sub-ranges (such as 1 to 3, 2 to 4). The same principle applies to a range with only one numerical value stated, such as "less than approximately 4.5", which should be interpreted to include the foregoing value and range. Further, regardless of the extent of the described range or the breadth of the characteristics, this interpretation should be applicable.

In embodiments, the methods for verifying endogenous coding of micropeptide OFMP include gene editing, immunoprecipitation and LC-MS/MS, specifically comprising:
using the gene editing method to correctly insert a Flag tag at the genomic location of micropeptide OFMP;
using the immunoprecipitation method in combination with chemiluminescence to observe the generation of a target band; and
using the LC-MS/MS method to detect the protein spectrum of micropeptide OFMP.

In embodiments, the gene indicators of the organ fibrosis cell model were detected by the real-time fluorescence quantitative PCR method, specifically comprising:
inoculating human hepatic stellate cells LX-2, human non-small cell lung cancer cell line A549, and human renal proximal tubular cells HK2 to culture plates, and adding different concentrations of recombinant human TGFβ1 stimulating factor; and
continuously stimulating for 24 h and 48 h, respectively, then detecting the mRNA expression levels of genes Colla1 and α-SMA by the real-time fluorescence quantitative PCR method, and determining whether the cell model is successfully constructed.

In embodiments, a novel micropeptide OFMP was obtained by the solid-phase synthesis method and the quality of the synthetic peptide was inspected, specifically comprising:
synthesizing peptide resin, comprising washing the resin with DMF after deblocking in 6-hydroxypyridine/N,N-dimethylformamide (DMF) deblocking liquid, then adding a condensing agent to fully react in a reactor, and lastly, thoroughly washing the resin with DMF to remove by-products;
adding a lysis buffer to fully lyse the polypeptide, and then separating the resin from the polypeptide using a sand-core funnel;
adding anhydrous ether to precipitate the polypeptide, centrifuging, discarding the supernatant, then washing the polypeptide with anhydrous ether, and vacuum drying the polypeptide to obtain crude polypeptide;
dissolving the crude polypeptide in deionized water, filtering it, and purifying it by semi-preparative high-performance liquid chromatography; and
detecting the purity of the polypeptide by analytical RP-HPLC.

In embodiments, the method for detecting the inhibitory effect of micropeptide OFMP in a fibrotic cell model by the real-time fluorescence quantitative PCR method comprises:
counting and inoculating human hepatic stellate cells LX-2, human non-small cell lung cancer cell line A549, and human renal proximal tubular cells HK2 to culture plates;
except for the control group, adding 10nmol of recombinant human TGFβ1 stimulating factor to stimulate continuously for 24 h;
extracting and collecting the total RNA of the cell samples with a TRIzol reagent, and then reverse-transcribing the extracted total RNA using a reverse transcription reagent, and performing qPCR reaction using the qPCR premix solution; and
detecting mRNA expression levels of genes Colla1 and α-SMA using a real-time fluorescence quantitative PCR instrument.

### Embodiment 1

In this embodiment, the expression levels of genes ENSG00000224023, ENSG00000263126 and ENSG00000265666 were analyzed in various organ fibrosis cases.

### (1) Analysis of OFMP expression level

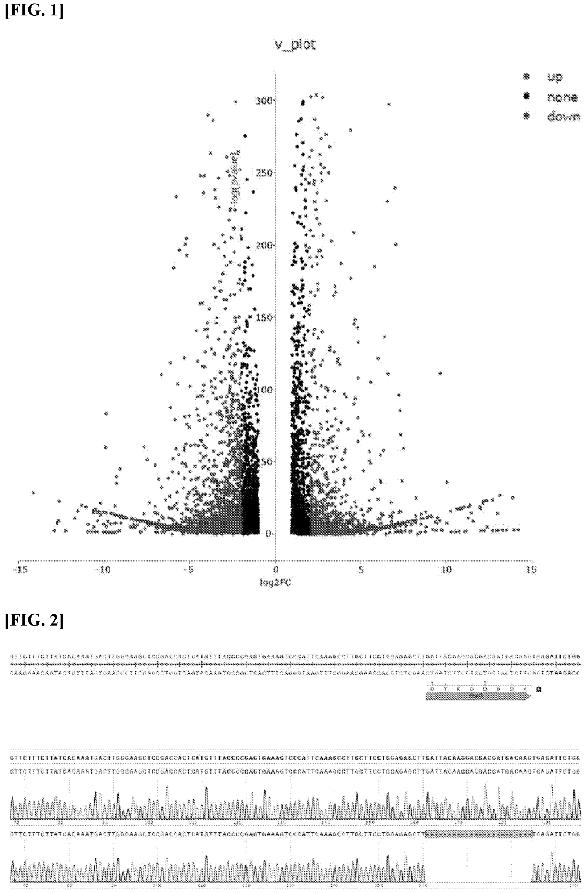

By using keywords "fibrosis" and "human intelligence", a search was conducted in the NCBI database, and multiple types of high-throughput sequencing data related to organ fibrosis were downloaded in batches, including human liver fibrosis and normal cell data (accession number: PRJNA638727), human liver fibrosis patient and healthy tissue data (accession number: PRJEB27201), human renal fibrosis and normal cell data (accession number: GSE23338), and human pulmonary fibrosis patient and healthy tissue data (accession number: PRJNA878759). Subsequently, the downloaded sequencing data was processed and analyzed. Finally, the number of reads with a comparison success rate greater than 90% was selected as the data for subsequent analysis. Then, differential gene expression analysis was conducted using the DESeq2 package (v1.38.3). The results showed that the significance padj of ENSG00000224023, ENSG00000263126 and ENSG00000265666 was less than 0.05 and the fold change (log2FoldChange) was less than -1, indicating that these genes were expressed at a low level in various organ fibrosis cases. The expression differences are shown in FIG. 1, with green representing low expression.

### (2) Detection of OFMP expression level

First, the RNA extraction reagent TRIzol was used to extract the total RNA of the organ fibrosis samples. Then, the purity and concentration of the extracted RNA was quantified by a NanoDrop ultra micro spectrophotometer, and the integrity of the extracted RNA was inspected by agarose gel electrophoresis. Subsequently, the RT-PCR reagent HiScript^{®} III RT SuperMix and the qPCR reagent 2×ChamQ SYBR qPCR Master Mix were used to perform reverse transcription and fluorescence quantification of the total RNA extracted from the fibrotic samples, thereby detecting the expression level of OFMP. The reaction system is as shown in Table 1:

**Table 1 Reaction system for detecting the expression level of OFMP**

| Reagent | Dose (µl) |
|---|---|
| 2 × ChamQ SYBR qPCR Master Mix | 10.0 |
| Primer F | 0.5 |
| Primer R | 0.5 |
| cDNA | 1.0 |
| ddH₂O | 8 |
| Total | 20 |

After the above components were mixed well, the following procedure proceeded: pre-denaturation at 95°C for 30s, 95°C for 10s, 60°C for 30s, 50 cycles was followed. After the qPCR reaction is completed, the Cq value of each sample well was collected. The maximum error of the Cq values among the three duplicate wells of each sample should be no more than 0.5 to ensure data accuracy. At the same time, the melting curve of the designed primers was observed to determine whether it was a single peak to judge the specificity of the reaction. The primers F and R involved in this reaction system are as follows:
(a) F: AGTGCCCGGACTTTTGTTCT; R: TGGGACTTTCACTCGGGGTA;
(b) F: ACCACATGGGCTCCGTTATG; R: GCTGCTTTGCCAAGTGAGTT;
(c) F: AGACACCGGCTTGTGTCATT; R: TCTGCCTGTGTTTGTGCCA.
(a) - (c) are the primer pairs designed for detecting ENSG00000224023, ENSG00000263126, ENSG00000265666 or their ORFs or corresponding micropeptides. For example, (a) can be used to detect ENSG00000224023, of ORF of ENSG00000224023, or the corresponding micropeptide OFMP3178.

### Embodiment 2

In this embodiment, the coding ability of the micropeptide OFMP was subjected to endogenous detection, including gene editing, immunoprecipitation and LC-MS/MS.

The gene sequences of ENSG00000224023, ENSG00000263126 and ENSG00000265666 were downloaded from the Ensembl database. Corresponding gene editing vectors were designed and constructed, and imported into 293T cells. The cells were digested and collected by trypsin. After centrifuging and discarding the supernatant, the cells were resuspended and subjected to monoclonal inoculation to culture plates and sequencing. Monoclonal cells that were correctly inserted with Flag tags were screened, as shown in FIG. 2-FIG. 4.

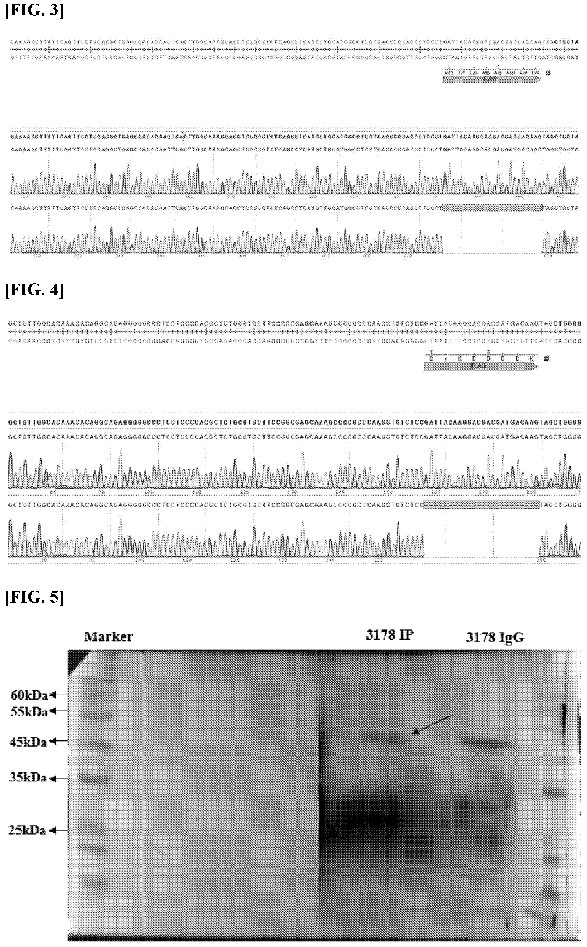

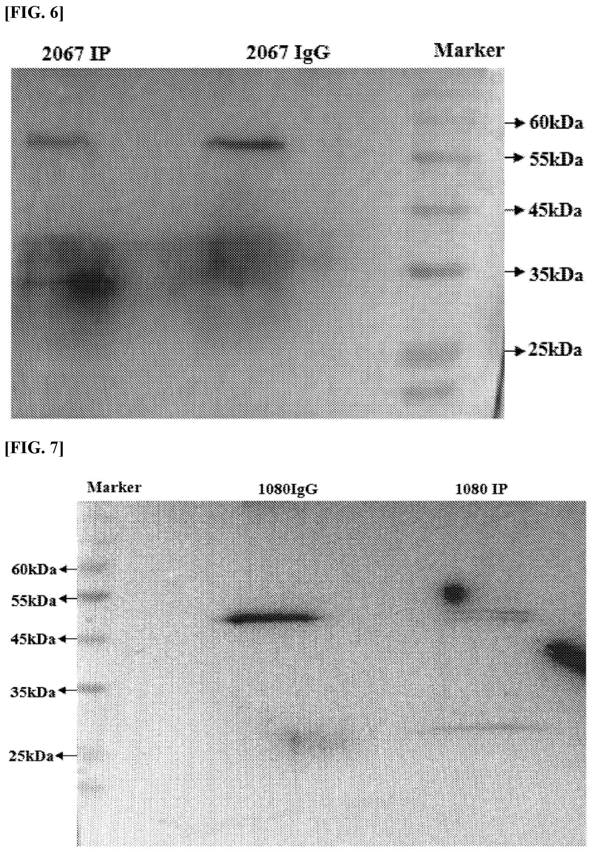

The cells were rinsed with PBS, digested and collected by trypsin, and centrifuged. After the supernatant was discarded, the cells were resuspended in an RIPA lysis buffer for precipitation, lysed on ice for 30 min, and centrifuged at 12,000 g for 15 min. The supernatant was collected, and 10 µl of antibody gel was added to form a protein-antibody-gel complex for incubation. After centrifuging at 6,000 g for 5 min, 1×SDS loading buffer was added, mixed well, and boiled up for denaturation for 10 min. The total protein was separated by 12% SDS-PAGE gel, and transferred to a PVDF membrane, then was added with 5% skimmed milk for blocking at room temperature for 1 h, and then washed with TBST for five times, 5 min each time. The first antibody of Flag was added at 4°C to incubate overnight and then it was washed with TBST 5 times, 5 min each time. The second antibody was inoculated at room temperature for 1 h and then it was washed with TBST 5 times, 5 min each time. An enhanced chemiluminescent (ECL) solution was added for staining. A Tannon imaging system was used to detect whether the target band existed. The results are shown in FIG. 5-FIG. 7.

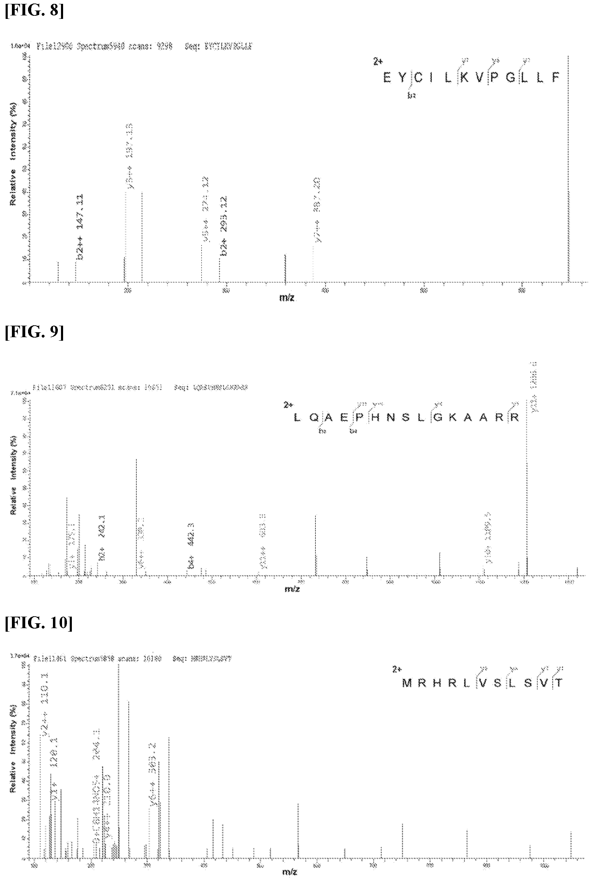

In order to further demonstrate the coding capability of micropeptide OFMP, the SDS-PAGE gel containing the target protein in the above steps was stained with Coomassie Brilliant Blue and then subjected to LC-MS/MS (liquid chromatography tandem quadrupole mass spectrometry) analysis, resulting in the mass spectrometry results of the target micropeptide, as shown in FIG. 8-FIG. 10.

After gene editing, immunoprecipitation and mass spectrometry analysis, it was found that after the Flag tags were inserted into the genomic sequence of the target micropeptide, the target band of micropeptide OFMP and the mass spectrometry peptide segment appeared, fully demonstrating that the novel micropeptide OFMP was capable of endogenous coding. ENSG00000224023, ENSG00000263126 and ENSG00000265666 coded micropeptide OFMP3178, OFMP2067 and OFMP1080, respectively. The amino acid sequences of the micropeptides are shown in SEQ ID NO: 1-3, respectively.

### Embodiment 3

In the embodiment, the gene indicators of 3 organ fibrosis cell models were detected by the real-time fluorescence quantitative PCR method.

### (1) Primer design

The key indicators of the organ fibrosis cell models include a collagen type I αl chain gene (Colla1), a transforming growth factor β1 (TGFβ1), a connective tissue growth factor (CTGF), and α-smooth muscle actin (a-SMA). The corresponding primers were designed using Primer Premier 5.0 as follows:
Colla1 upstream and downstream primer pairs:
F: ACTGGTGAGACCTGCGTGTA, R: AATCCATCGGTCATGCTCTC;
α-SMA upstream and downstream primer pairs:
F: ACGAGACCACCTACAACAGCAT, R: CTCGTCG
   TACTCCTGCTTGGT;
TGFβ1 upstream and downstream primer pairs:
   F: AAGTGGACATCAACGGGTTC, R: GGTCCTTGCGGAAGTCAATG;
CTGF upstream and downstream primer pairs:
   F: GGAAGAGAACATTAAGAAGGGCAA, R: CTCGGTATGTCTTCATGCTGGTG.

According to the TRIzol reagent manual of Life Invitrogen, the total RNA of the cell samples was extracted and collected. Then, the purity and concentration of the extracted RNA was quantified by a NanoDrop 2000 ultra micro spectrophotometer. The integrity of the extracted RNA was inspected and ensured by agarose gel electrophoresis. A reverse transcription reagent HiScript^{®} III RT SuperMix for qPCR (+ genomic DNA wiper) was used to perform reverse transcription on the extracted total RNA and a qPCR premix liquid ChamQ SYBR qPCR Master Mix was used to perform qPCR reaction. The reaction system is shown in Table 2:

**Table 2 qPCR system**

| Reagent | Dose (µL) |
|---|---|
| 2 × ChamQ SYBR qPCR Master Mix | 10.0 |
| Primer F | 0.4 |
| Primer R | 0.4 |
| cDNA | 1.0 |
| ddH₂O | 8.2 |
| Total | 20 |

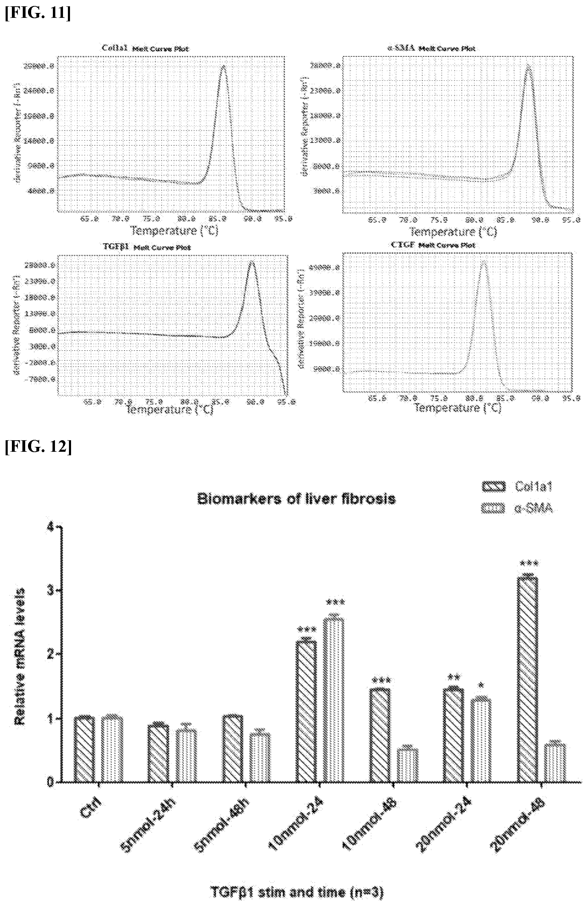

After the above components were mixed well, the following procedure proceeded: initial denaturation 95°C 30s, 95°C 10s, 60°C 30s, 50 cycles. After the qPCR reaction was completed, the Cq value of each sample well was collected. The maximum error of the Cq values among the three duplicate wells of each sample should be no more than 0.5 to ensure data accuracy. At the same time, the melting curves of the designed primers were observed to determine whether it was a single peak to judge the specificity of the reaction, as shown in FIG. 11.

### (2) Model construction

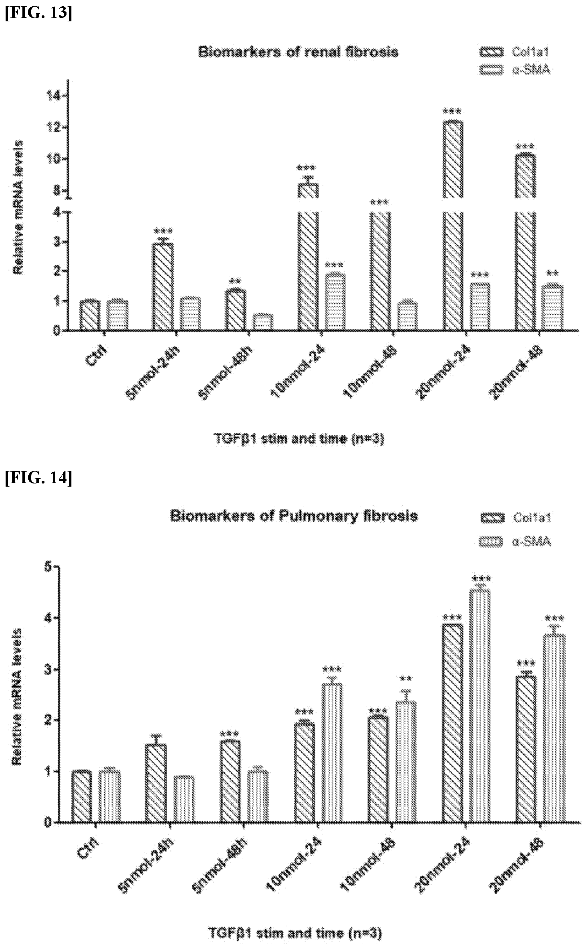

Human hepatic stellate cells LX-2, human non-small cell lung cancer cell line A549, and human renal proximal tubular cells HK2 were cultured in a 37°C 5% CO₂ cell incubator until the cell density was above 80%, and the cells were digested and collected by trypsin, and centrifuged. After the supernatant was discarded, the cells were counted by a cell counter, inoculated to six-well cell culture plates, and put into a 37°C 5% CO₂ cell incubator to continue the culturing. The culture medium was sucked out after the cells were cultured for 24 h, different concentrations of recombinant human TGF β 1 stimulating factor were added to stimulate continuously for 24 h, 48 h, and the mRNA expression levels of genes Colla1 and α-SMA were detected by the qPCR method, as shown in FIG. 12, FIG. 13 and FIG. 14.

### Embodiment 4

In this embodiment, a micropeptide OFMP was obtained by the solid-phase synthesis method and the quality of the synthetic micropeptide was inspected.

In this embodiment, a micropeptide OFMP was synthesized by the solid-phase peptide synthesis method, the synthetic micropeptide OFMP was separated and purified by preparative HPLC, and the purity of the micropeptide OFMP was determined by analytical RP-HPLC. In the solid-phase peptide synthesis method, Fmoc-Thr-wang-resin was used as the starting material, and then protective amino acids were successively coupled to the last amino acid of the target sequence. After the coupling work was completed, thorough washing, lysis, and post-treatment were conducted to obtain a crude polypeptide product. After the crude product was dissolved, it was purified, concentrated and freeze-dried using preparative high-performance liquid chromatography to obtain a pure polypeptide product. The specific reaction steps are as follows:
(1) Peptide resin synthesis

Weigh 1 mmol of Fmoc-Thr-wang resin, pour it into a glass sand-core reaction column, and add 20 mL of CH₂Cl₂ to fully expand the resin.
a. Deblocking: Add 20mL of 20% 6-hydroxypyridine/N,N-dimethylformamide (DMF) deblocking liquid, react for a certain period, then suck out the deblocking liquid, wash the resin with DMF once, and add 20 mL of the deblocking liquid again for reaction to remove the Fmoc protecting group;
b.Washing: suck out the deblocking liquid, and wash the resin with DMF to thoroughly remove by-products;
c.Condensation: dissolve the protecting amino acid and activator used for coupling in DMF and a condensing agent, and then react thoroughly in a reactor;
d.Washing: suck out the deblocking liquid, and wash the resin with DMF to thoroughly remove by-products.

### (2) Lysis

Put the drained resin into a conical flask, add a lysis buffer to fully lyse the synthetic polypeptide, and then separate the resin from the polypeptide using a sand-core funnel. The composition of the lysis buffer and the volume ratio of each component are as follows: trifluoroacetic acid: phenol: water: thioanisole: EDT = 90:3:3:2:2.

### (3) Post-processing

Add the lysis buffer to anhydrous ether that has experienced an ice bath, slowly stir to precipitate the polypeptide, centrifuge and discard the supernatant, wash the polypeptide with anhydrous ether and suck out the anhydrous ether to obtain a crude polypeptide product.

### (4) Purification

a.Dissolution: dissolve the crude polypeptide product in deionized water and filter it through a filter membrane with a pore size of 0.45 µm.
b.Preparation: Purify through semi-preparative high-performance liquid chromatography, with the mobile phase: A being 0.1% TFA (acetonitrile), B being 0.1% TFA (water). The purification system is shown in Table 3.

**Table 3 Polypeptide purification system**

| Time (min) | Flow rate (mL/min) | A% | B% | Wavelength nm |
|---|---|---|---|---|
| 0 | 10 | 15 | 85 | 220 |
| 50 | 10 | 50 | 50 | 220 |

Collect the solution at an ultraviolet wavelength of 220nm, confirm by MS that the molecular weight was consistent with the theoretical value, and analyze the purity of this solution by HPLC, which was above 95%. Concentrate the qualified solution under vacuum at 37°C by a rotary evaporator to remove the organic solvent, put it in a freeze-drying tray and freeze it at -80°C, and freeze-dry it in a freeze-drying machine to obtain a pure polypeptide product.

### (5) Purity detection

Collect the purified product after freeze-drying, and detect polypeptide purity using analytical RP-HPLC. Analytical conditions: mobile phase: 0.1% TFA (acetonitrile), 0.1% TFA (water); loading volume: 10µL. The specific reaction system is shown in Table 4:

**Table 4 Purity detection system**

| Time (min) | Flow rate (mL/min) | A% | B% | Wavelength nm |
|---|---|---|---|---|
| 0.0 | 1 | 20/15/6 | 80/85/94 | 220 |
| 25.0 | 1 | 75/70/50 | 25/30/50 | 220 |
| 25.1 | 1 | 100 | 0 | 220 |
| 30.0 | 1 | Stop | | 220 |

| | | | | |
|---|---|---|---|---|
| Note: In the table, the experimental parameters of OFMP3178, OFMP2067, and OFMP1080 are listed in sequence. | | | | |

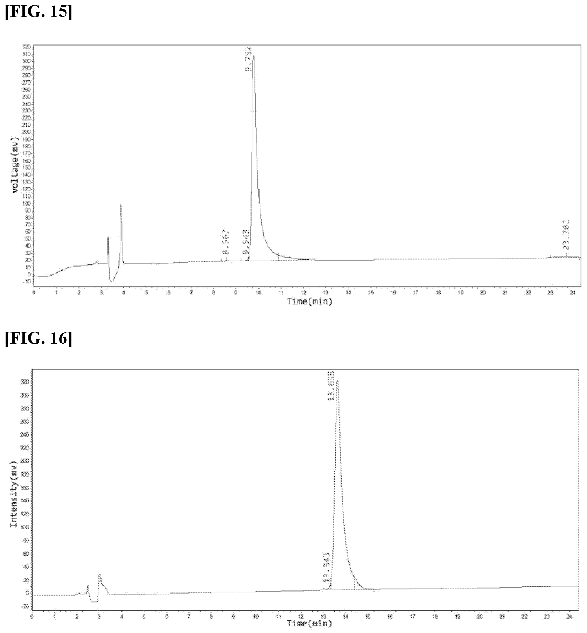

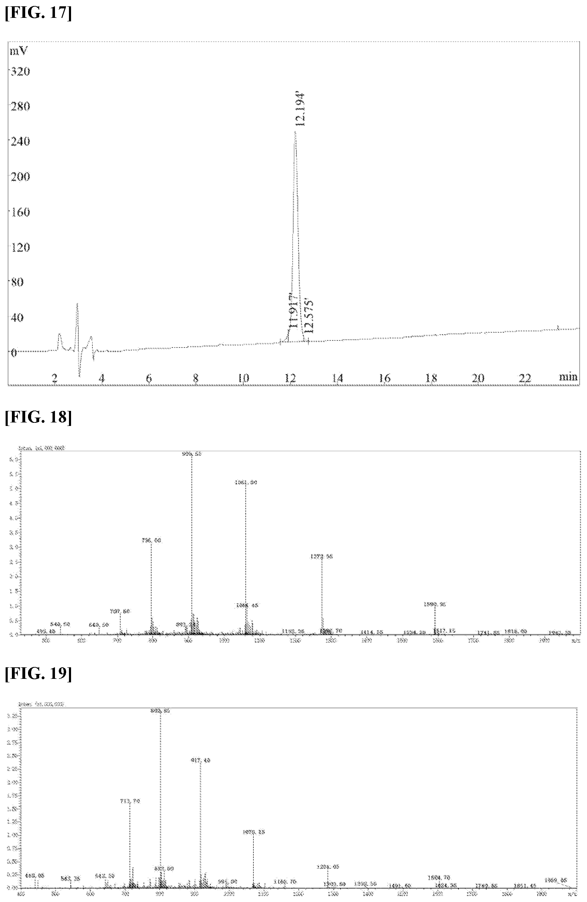

The purity of the synthetic polypeptide was detected by reversed-phase liquid chromatography. The purity detection results are shown in Table 5, indicating that the purity of each of the prepared micropeptide OFMP was greater than 95%, as shown in FIG. 15-FIG. 20, which meets the requirements of the subsequent experiments.

**Table 5 Purity detection results**

| Name | Peak Area | Purity (%) |
|---|---|---|
| Micropeptide OFMP3178 | 5510656 | 95.31 |
| Micropeptide OFMP2067 | 7344369 | 96.03 |
| Micropeptide OFMP1080 | 3505504 | 97.89 |

### Embodiment 5

In this embodiment, the inhibitory effect of micropeptide OFMP on the human liver fibrosis cell model was detected.

Human hepatic stellate cells LX-2, human non-small cell lung cancer cell line A549, and human renal proximal tubular cells HK2 were cultured in a 37°C 5% CO₂ cell incubator until the cell density was above 80%, and the cells were digested and collected by trypsin, and centrifuged. After the supernatant was discarded, the cells were counted by a cell counter, inoculated to six-well cell culture plates, and put into a 37°C 5% CO₂ cell incubator to continue the culturing. The culture medium was sucked out after the cells were cultured for 24 h, a 2‰ DMSO solvent was added as a negative control, 20µmol of hydronidone was added as a positive control, 40µmol, 10µmol, 2.5µmol, 625nmol and 156nmol of the synthetic peptide OFMP were added in the equal proportion for other groups as administration groups, 10nmol of recombinant human TGFβ1 stimulating factor was added to other groups except the control groups half an hour later to continuously stimulate for 24 h, and the mRNA expression levels of genes Colla1, TGFβ1 and CTGF in OFMP3178, and the mRNA expression levels of genes Colla1 and α-SMA in OFMP2067 and OFMP1080 were detected.

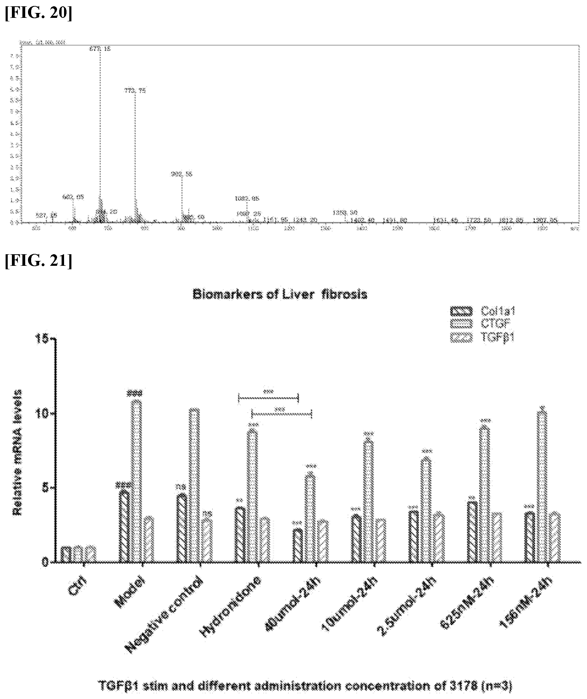

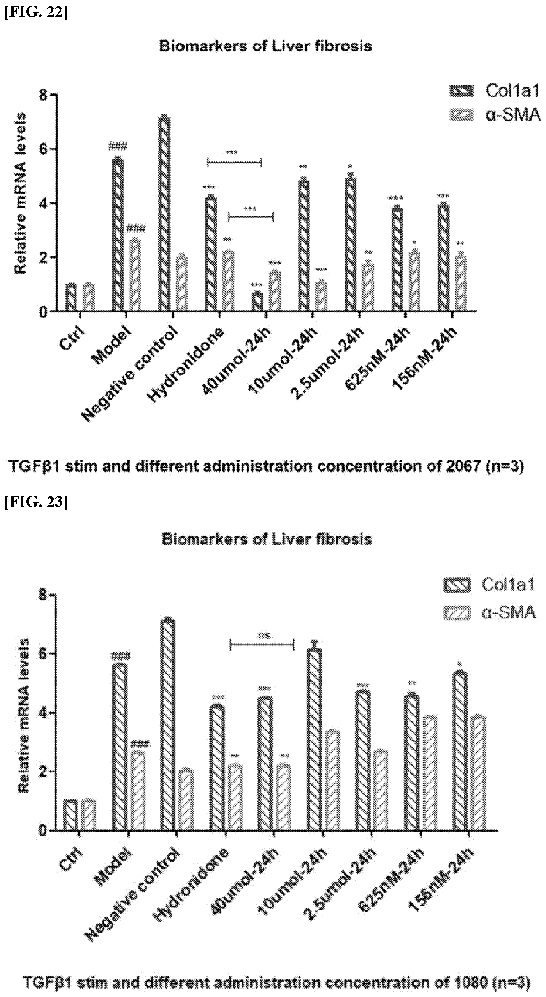

The results are shown in FIG. 21-FIG. 23. Compared with the model group, after administration of different concentrations of the synthetic peptide OFMP3178, the expression levels of the genes Colla1 and CTGF in liver fibrosis cells significantly decreased. Under the treatment condition of 40µmol-24h, the expression levels of the genes Colla1 and CTGF in the liver fibrosis cells showed the most significant decrease. Moreover, there was a significant difference from the positive drug hydronidone group. The results showed that the synthetic peptide OFMP3178 had a good therapeutic effect on liver fibrosis.

Compared with the model group, after administration of different concentrations of the synthetic peptide OFMP2067, the expression levels of the genes Colla1 and α-SMA in liver fibrosis cells significantly decreased. Under the treatment condition of 40µmol-24h, the expression levels of the genes Colla1 and α-SMA in the liver fibrosis cells showed the most significant decrease. Moreover, there was a significant difference from the positive drug hydronidone group. The results showed that the synthetic peptide OFMP2067 had a good therapeutic effect on liver fibrosis.

Compared with the model group, after administration of different concentrations of the synthetic peptide OFMP1080, the expression levels of the gene Colla1 in liver fibrosis cells significantly decreased. Under the treatment condition of 40µmol-24h, the expression levels of the genes Colla1 and α-SMA in the liver fibrosis cells showed the most significant decrease. Moreover, there was not a significant difference from the positive drug hydronidone group. The results showed that the synthetic peptide OFMP1080 had a good therapeutic effect on liver fibrosis.

### Embodiment 6

In this embodiment, the inhibitory effect of micropeptide OFMP on the human renal fibrosis cell model was detected.

Human renal proximal tubular cells HK2 were cultured in a 37°C 5% CO₂ cell incubator until the cell density was above 80%, and the cells were digested and collected by trypsin, and centrifuged. After the supernatant was discarded, the cells were counted by a cell counter, inoculated to six-well cell culture plates, and put into a 37°C 5% CO₂ cell incubator to continue the culturing. The culture medium was sucked out after the cells were cultured for 24 h, a 2‰ DMSO solvent was added as a negative control, 20µmol of hydronidone was added as a positive control, 40µmol, 10µmol, 2.5µmol, 625nmol and 156nmol of the synthetic peptide OFMP were added in the equal proportion for other groups as administration groups, 10nmol of recombinant human TGFβ1 stimulating factor was added to other groups except the control groups half an hour later to continuously stimulate for 24 h, and the mRNA expression levels of genes Colla1, TGFβ1 and CTGF in OFMP3178, and the mRNA expression levels of genes Colla1 and α-SMA in OFMP2067 and OFMP1080 were detected.

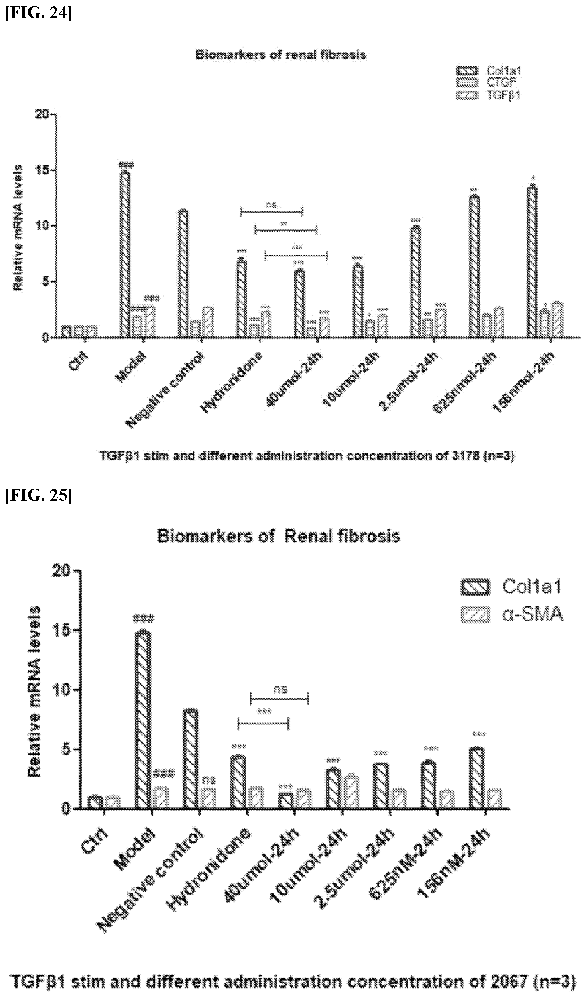

The results are shown in FIG. 24-FIG. 26. Compared with the model group, after administration of different concentrations of the synthetic peptide OFMP3178, the expression levels of the genes Colla1, CTGF and TGFβ1 in renal fibrosis cells significantly decreased. Under the treatment condition of 40µmol-24h, the expression levels of the genes Colla1, CTGF and TGFβ1 in the renal fibrosis cells showed the most significant decrease. Moreover, there was a significant difference from the positive drug hydronidone group. The results showed that the synthetic peptide OFMP3178 had a good therapeutic effect on renal fibrosis.

Compared with the model group, after administration of different concentrations of the synthetic peptide OFMP2067, the expression levels of the genes Colla1 and α-SMA in renal fibrosis cells significantly decreased. Under the treatment condition of 40µmol-24h, the expression levels of the genes Colla1 and α-SMA in the renal fibrosis cells showed the most significant decrease. Moreover, there was a significant difference from the positive drug hydronidone group. The results showed that the synthetic peptide OFMP2067 had a good therapeutic effect on renal fibrosis.

Compared with the model group, after administration of different concentrations of the synthetic peptide OFMP1080, the expression levels of the genes Colla1 in renal fibrosis cells significantly decreased. Under the treatment condition of 40µmol-24h, the expression levels of the genes Colla1 in the renal fibrosis cells showed the most significant decrease. The results showed that the synthetic peptide OFMP1080 had a good therapeutic effect on renal fibrosis.

### Embodiment 7

In this embodiment, the inhibitory effect of micropeptide OFMP on the human pulmonary fibrosis cell model was detected.

Human non-small cell lung cancer cell line A549 was cultured in a 37°C 5% CO₂ cell incubator until the cell density was above 80%, and the cells were digested and collected by trypsin, and centrifuged. After the supernatant was discarded, the cells were counted by a cell counter, inoculated to six-well cell culture plates, and put into a 37°C 5% CO₂ cell incubator to continue the culturing. The culture medium was sucked out after the cells were cultured for 24 h, a 2‰ DMSO solvent was added as a negative control, 2µmol of Nintedanib was added as a positive control, 40µmol, 10µmol, 2.5µmol, 625nmol and 156nmol of the synthetic peptide OFMP were added in the equal proportion for other groups as administration groups, 10nmol of recombinant human TGFβ1 stimulating factor was added to other groups except the control groups half an hour later to continuously stimulate for 24 h, and the mRNA expression levels of genes Colla1, TGFβ1 and CTGF in OFMP3178, and the mRNA expression levels of genes Colla1 and α-SMA in OFMP2067 and OFMP1080 were detected.

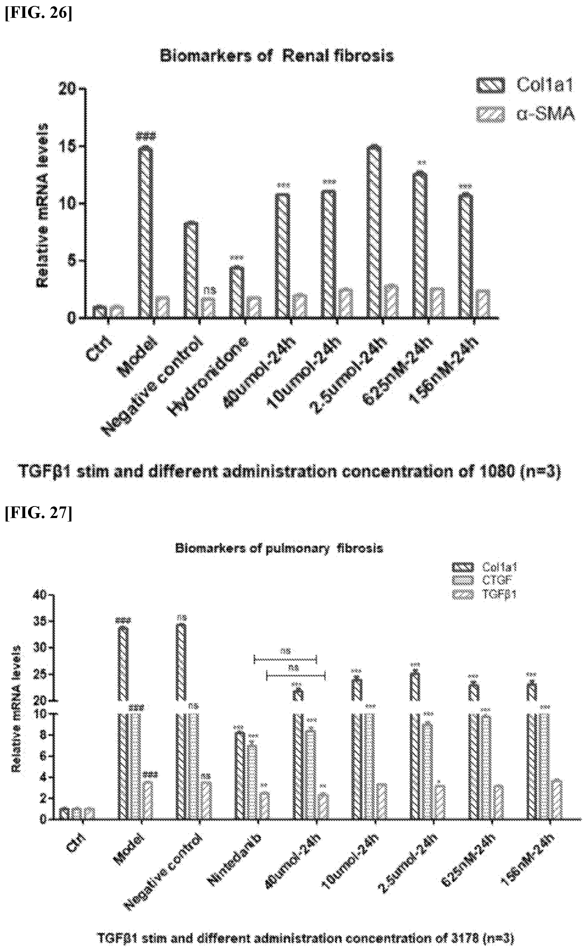

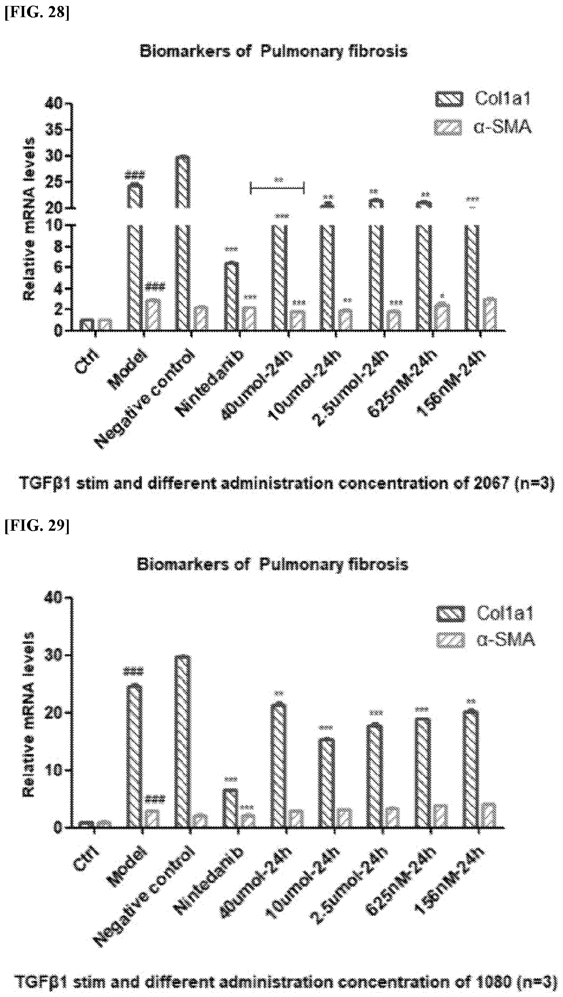

The results are shown in FIG. 27-FIG. 29. Compared with the model group, after administration of different concentrations of the synthetic peptide OFMP3178, the expression levels of the genes Colla1, CTGF and TGFβ1 in pulmonary fibrosis cells significantly decreased. Under the treatment condition of 40µmol-24h, the expression levels of the genes Colla1, CTGF and TGFβ1 in the pulmonary fibrosis cells showed the most significant decrease. Moreover, there was a significant difference from the positive drug Nintedanib group. The results showed that the synthetic peptide OFMP3178 had a good therapeutic effect on pulmonary fibrosis.

Compared with the model group, after administration of different concentrations of the synthetic peptide OFMP2067, the expression levels of the genes Colla1 and α-SMA in pulmonary fibrosis cells significantly decreased. Under the treatment condition of 40µmol-24h, the expression levels of the genes Colla1 and α-SMA in the pulmonary fibrosis cells showed the most significant decrease. Moreover, there was a significant difference from the positive drug Nintedanib group. The results showed that the synthetic peptide OFMP2067 had a good therapeutic effect on pulmonary fibrosis.

Compared with the model group, after administration of different concentrations of the synthetic peptide OFMP1080, the expression levels of the genes Colla1 in pulmonary fibrosis cells significantly decreased. Under the treatment condition of 10µmol-24h, the expression levels of the genes Colla1 in the pulmonary fibrosis cells showed the most significant decrease. The results showed that the synthetic peptide OFMP1080 had a good therapeutic effect on pulmonary fibrosis.

### Embodiment 8

In this embodiment, the antigen design, animal immunization, subcloning and Elisa test for quality control were carried out for the monoclonal antibody of micropeptide OFMP.

Corresponding antigen sequences were designed based on the sequences of micropeptides OFMP3178, OFMP2067 and OFMP1080, antigen synthesis and coupling were completed, and the foregoing antigens were used for four regular immunizations of four healthy female BALB/c mice (mouse age 8-12 weeks). After the last immunization injection, blood was drawn to detect antiserum Elisa titer (polypeptide antigen coating plate), and the mice with an antiserum titer of greater than 8K were selected to proceed to the fusion stage. After completion of the fusion, the cells were diluted and inoculated to a 384-well plate for 14 days of culture, then the supernatant in the plate was taken for Elisa detection, and for antigen reaction, the cells with high affinity were selected for subcloning.

The selected cells were inoculated to a 96-well plate, Elisa was used to detect the reaction of the supernatant of the well plate with the antigen, and the cells with high affinity were selected for next round of subcloning. The above steps were repeated until the positive rate of the cell strains in the plate reached 100%, i.e., obtaining the monoclonal antibody and successfully establishing the cell strains. Elisa detection was performed for the corresponding antigen, with the maximum protein antigen coating of 10 ng, the coating was diluted till the minimum coating of 0.02 ng, and 5% milk PBST was used for blocking. The dilution factor of the first antibody was 1:1000, and it was incubated at 37°C for 1 h; the second antibody was incubated at 37°C for 45 min; a staining solution was added and reacted at 37°C for 15 min, and then the reaction was terminated and the results were read. The results are shown in Table 6.

**Table 6: Elisa sensitivity test of monoclonal antibodies of micropeptide OFMP**

| Antibody's Name | 10ng | 0.15625ng | 0.019531ng | PC | NC | Titer |
|---|---|---|---|---|---|---|
| Micropeptide OFMP3178 | 1.528 | 0.298 | 0.153 | 2.91 | 0.06 | 0.15625ng |
| Micropeptide OFMP2067 | 1.873 | 0.318 | 0.222 | 2.91 | 0.06 | 0.078125ng |
| Micropeptide OFMP1080 | 2.439 | 0.282 | 0.236 | 3.52 | 0.07 | 0.078125ng |

This embodiment indicates that the monoclonal antibodies of OFMP can be used to detect micropeptide OFMP.

## Claims

1. An organ fibrosis-related micropeptide (OFMP), wherein the amino acid sequence of the micropeptide is any of the following ones:
(a) an amino acid sequence shown in any of SEQ ID NO: 1-3;
(b) an amino acid sequence that has a homology of more than 85% with the amino acid sequence shown in any of SEQ ID NO: 1-3;
(c) an amino acid sequence formed by replacing, deleting or adding one or several amino acid residues in the amino acid sequence shown in any of SEQ ID NO: 1-3, which has the same function as the original sequence.

2. A nucleotide sequence coding the micropeptide described in claim 1.

3. A use of the micropeptide described in any one of claims 1-2 in preparing reagents or drugs for detecting or treating organ fibrosis.

4. A use of long non-coding RNA (lncRNA) in preparing reagents or drugs for detecting or treating organ fibrosis, wherein the ID number of the lncRNA in the Ensembl database is: ENSG00000224023, ENSG00000263126 or ENSG00000265666.

5. The use according to claim 3 or 4, wherein the organ fibrosis includes one or more of liver fibrosis, renal fibrosis, pulmonary fibrosis, heart fibrosis, and skin fibrosis..

6. The use according to claim 3 or 4, wherein the detection of organ fibrosis is achieved through specific primers designed for the nucleotide sequences described in claim 2 or 4.

7. A recombinant vector containing the nucleotide sequence described in claim 2 or 4.

8. A pharmaceutical composition, wherein the pharmaceutical composition contains the micropeptide described in claim 1, the nucleotide sequence described in claim 2 or the nucleotide sequence described in claim 4, and a pharmaceutically acceptable carrier thereof.

9. An organ fibrosis detection kit, wherein the kit contains specific primer pairs and/or antibodies designed for the nucleotide sequence described in claim 2 or 4.

10. The organ fibrosis detection kit according to claim 9, wherein the nucleotide sequences of the specific primer pairs are selected from any one of the following groups:
(a) F: AGTGCCCGGACTTTTGTTCT; R: TGGGACTTTCACTCGGGGTA;
(b) F: ACCACATGGGCTCCGTTATG; R: GCTGCTTTGCCAAGTGAGTT;
(c) F: AGACACCGGCTTGTGTCATT; R: TCTGCCTGTGTTTGTGCCA.
